# FASCICULE DE BREVET EUROPEEN

(11) **EP 0 475 986 B1**
(45) Date de publication et mention de la délivrance du brevet: **01.12.1993**
(21) Numéro de dépôt: 90908546.6
(22) Date de dépôt: 25.05.1990
(51) Int. Cl.: G01N 33/24

(54) **PROCEDE ET DISPOSITIF DE MESURE IN SITU DES CARACTERISTIQUES DE GONFLEMENT D'UN SOL**
VERFAHREN UND GERÄT ZUR MESSUNG VOR ORT DER QUELLCHARAKTERISTIK VON BÖDEN
METHOD AND DEVICE FOR IN-SITU MEASUREMENT OF GROUND HEAVE CHARACTERISTICS

(30) Priorité: 09.06.1989 FR 8907924
(43) Date de publication de la demande: 25.03.1992
(73) Titulaire: E.R.G., F-83500 La Seyne-sur-Mer (FR)
(72) Inventeur: MUSCHOTTI, Ernest, F-83500 La Seyne-sur-Mer (FR); FLAVIGNY, Etienne, F-38100 Grenoble (FR)
(74) Mandataire: Moretti, René
(86) Numéro de dépôt international: FR9000368
(87) Numéro de publication internationale: WO9015324

(56) Documents cités:
- FR-A- 1 586 243
- FR-A- 2 512 860
- GB-A- 817 295
- US-A- 4 420 975

## Description

La présente invention a pour objet des procédés et des dispositifs de mesure in situ des caractéristiques de gonflement d'un sol.

Le secteur technique de l'invention est la fabrication de matériel et d'appareillage pour effectuer des essais et des mesures de mécanique des sols in situ.

Une des applications principales de l'invention est la détermination de la capacité de gonflement d'un sol préalablement à la construction d'un ouvrage sur celui-ci.

En effet, de nombreux ouvrages, maisons d'habitation de faible hauteur ou implantations industrielles ont eu à subir des dommages du fait des sols expansifs, et spécialement des sols gonflants.

En FRANCE, plusieurs cas de désordres ont été répertoriés dans la région parisienne par Monsieur PHILIPPONNAT. D'autres exemples sont cités en ROUMANIE (Monsieur POPESCU), au SENEGAL (Monsieur THUREAU) ou aux ETATS-UNIS (Monsieur CHEN) par exemple. On peut remarquer que ces problèmes se présentent toujours dans des sols argileux, sous des climats arides ou semi-arides, c'est-à-dire en milieu non saturé.

Ces sols argileux expansifs provoquent de graves problèmes aux constructions, et ce d'autant plus que celles-ci sont légères. De plus, ces désordres s'amplifient avec le temps, de manière très lente, au fur et à mesure de la perte de rigidité des ouvrages. Les désordres les plus courants dus aux sols gonflants sont des déformations différentielles, ainsi que des fissurations s'ouvrant et se refermant au rythme des saisons et donc du cycle retrait-gonflement du sol argileux.

Ce sont des désordres de ce genre qui conduisent parfois à la ruine de certaines constructions. Les parties les plus exposées à l'action des sols gonflants sont les fondations et de nombreuses dispositions constructives sont préconisées, en vue de leur protection, mais sans optimisation par manque de mesure et de connaissance de la valeur et du risque encouru, ce qui coûte cher quand on veut être sûr d'éviter tout risque.

On peut utiliser en particulier :
- un traitement préventif du sol de fondation;
- une adaptation des structures;
- un drainage périphérique en vue d'obtenir un équilibre hydrique du sol.

Ces dispositions préventives coûtant cher par manque d'optimisation sont alors souvent plutôt ignorées.

Il apparaît donc nécessaire d'arriver à prévenir le gonflement des sols de fondation, et donc d'être capable de déterminer par un essai soit in situ, soit de laboratoire la propension au gonflement futur de l'argile constituant le sol à étudier.

A ce jour en effet, il n'est pas possible de connaître le comportement d'un sol sans effectuer des essais sur un échantillon de celui-ci ou sur place : les mécanismes de réaction d'un sol sont en effet très complexes et sont liés à sa structure interne qui est souvent très hétérogène et non répétitive. Ainsi, on peut classer les sols par catégorie mais sans pouvoir en quantifier les caractéristiques en dehors d'essai : de nombreuses publications, mesures et études ont été faites et sont faites sur chaque paramètre concerné par les problèmes du gonflement qui nous préoccupent, tels que, pour les sols non saturés, la succion dans les sols, le comportement hydrodynamique et l'écoulement de l'eau, la contrainte effective, la résistance au cisaillement etc ....

Divers appareils ont été développés pour effectuer des mesures de ces paramètres, sachant que, pour un paramètre donné, il existe une gamme d'appareillages, fonction souvent de la gamme de valeur à mesurer.

Cependant, il s'avère que le renseignement principal concernant l'étude des sols non saturés est celui de la valeurs de sa succion, et il existe une corrélation entre la baisse de celle-ci et l'augmentation du gonflement, laquelle corrélation est une des clés de la perception des mécanismes et des paramètres du gonflement.

Cette aptitude au gonflement d'un sol est uniquement étudiée à ce jour en laboratoire à base d'essais dit "oedométrique" sur échantillons.

La méthode la plus classique, à quelques variantes près, reste celle préconisée par PAREZ et BACHELIER qui empêche par un contrepoids asservi l'échantillon de gonfler et permet sans difficulté de déterminer la valeur après stabilisation, de la pression exercée, qui est la pression de gonflement.

En effet, la pression de gonflement d'un sol peut être définie soit :
- comme la pression qu'il faut appliquer à un sol pour qu'aucune variation de volume n'intervienne dans celui-ci lors de sa saturation;
- comme la pression qu'il faut exercer sur un sol pour le ramener après saturation à son volume initial avant saturation.

La première de ces deux définitions est la plus classique et reste celle employée par la plupart des auteurs.

Cependant, ces méthodes considèrent le problème de gonflement comme étant un phénomène unidirectionnel, alors que la mise en évidence de l'anisotropie du gonflement dans les sols non saturés devrait inciter à ne plus se limiter à une seule direction de gonflement, mais à l'étudier sur des appareils dérivant plus d'une cellule tri-axiale que d'un "oedomètre".

De plus, ces essais en laboratoire sont longs, chers, ne donnent pas une réponse en temps réel, retardent donc l'information et la décision et perturbent l'organisation d'un chantier. Par ailleurs, l'échantillon qui est véhiculé du site en laboratoire peut changer d'état en cours de transport et dans le temps, et les valeurs mesurées ne sont plus représentatives.

Ceci explique du reste que ces essais en laboratoire ne sont que peu réalisés pour les besoins d'un chantier, pour lequelsoit on fait alors l'impasse, soit on surprotège les risques ; ces essais sont utilisés surtout dans la recherche et les expertises, et ces essais sont du reste inimaginables à mettre en oeuvre sur le terrain.

Cependant, depuis plusieurs années, la majorité des études courantes de fondation sont effectuées en ayant recours exclusivement à des essais in situ comme moyen de paramétrer les calculs de fondation, à partir de pénétromètres par exemple ou des matériels commercialisés sous la marque "Pressiomètre" particulièrement. Il est donc utile et intéressant de pouvoir effectuer également des mesures de gonflement et si possible, en utilisant le maximum de moyens de mise en oeuvre qui seraient communs aux autres essais et mesures effectuées.

Divers équipements en effet, existent pour la mesure de pression et de cisaillement in situ dans un sol, et la mesure de la capacité de gonflement est aussi basée sur des mesures de pression, mais le procédé de mise en oeuvre de mesure et d'analyse est différent des méthodes connues existantes et fait l'objet de la présente invention.

On peut citer dans ce domaine divers brevets qui ont été déposés depuis plusieurs années, comme en particulier ceux de la Société MENARD, qui est le précurseur dans ce domaine depuis plus de 30 ans et est titulaire de la marque déposée désignant ce matériel, le "PRESSIOMETRE". Un de ses derniers brevets déposé le 12 Juin 1981 et publié le 18 Mars 1983 sous le NO. FR-A- 2.512.860, intitulé "Dispositif de commande de surface de type numérique pour essai de sols et de roches in situ avec sonde profonde" revendique essentiellement des moyens et des procédés de calcul à partir des mesures relevées pour obtenir des résultats sur le site, malgré les contraintes d'opération. Les appareils "PRESSIOMETRE" utilisés par cette société pour obtenir ces mesures, sont connus par ailleurs et comprennent :
- une sonde profonde comportant une cellule principale dilatable, gonflable par pression de liquide, et généralement encadrée de deux cellules de garde du même genre, qui sont, elles, sollicitées sous pression gazeuse et,
- relié à la sonde profonde par une tubulure mixte liquide-gaz, un dispositif de surface permettant de faire varier la ou les pressions, en même temps que de détecter les variations de volume de la cellule principale.

D'autres détails sur ces appareils sont disponibles dans l'ouvrage "THE PRESSUREMETER AND FOUNDATION ENGINEERING" par F. BAGNELIN. J.F. JEZEQUEL. D.H. SHIELDS. dans la série on Rock and Soil Mechanics, Vol. 2 (1974/77) NO. 4, Trans. Tech. Publications, Clansthal, Germany. 1978".

Par ailleurs, on peut également citer le brevet déposé le 21 Juin 1985 par la Société SOPENA, publié le 26 Décembre 1986 sous le NO. FR-A-2.585.876 et intitulé "Procédé et dispositif de mesure des caractéristiques de cisaillement d'un sol" : l'invention revendiquée permet, outre la mesure de pression radiale comme dans les appareils décrits précédemment de la société MENARD, de mesurer in situ la traction axiale applicable à la sonde et provoquant la rupture du sol par cisaillement, et cela grâce à une enveloppe élastique de cette sonde à l'extérieur de laquelle sont montées des coquilles de pression.

Ainsi, chacun de ces appareils fournit des résultats de mesures correspondant chacun à une caractéristique du sol et sont donc complémentaires les uns des autres suivant ce que l'on recherche.

Aucun cependant dans leur configuration connue et leur procédé d'utilisation ne permet de mesurer et de connaître la pression et la dilatation de gonflement d'un sol.

Le problème posé est donc, à partir de moyens existant de mise en oeuvre de matériel connu et disponible dans le domaine public pour la mesure de pression du sol in situ, d'adapter une sonde et un appareillage de surface pour mesurer les caractéristiques de gonflement du sol sur chantier.

Une solution au problème posé est un procédé de mesure in situ, utilisant une sonde dilatable, des moyens pour introduire cette sonde dans le sol, des moyens pour contrôler la pression radiale exercée sur le sol par la sonde et tel que :
- on introduit dans un forage réalisé dans le sol à étudier ladite sonde dilatable à la profondeur voulue et on réalise un essai pressiométrique normal connu, consistant à établir la courbe de variation de la pression de la sonde et donc dans le sol autour de celle-ci, en fonction du volume de dilatation de cette sonde et donc du volume comprimé du sol;
- on apporte dans ledit sol entourant au moins une partie de la sonde et à partir du point de remise du sol à son état initial, comme s'il n'y avait pas de trou de forage, un fluide qui imbibe le sol, sous une charge faible correspondant à quelques mètres de colonne de ce fluide;
- on contrôle simultanément le volume de ladite sonde afin qu'il reste constant en augmentant alors la pression dans celle-ci, jusqu'à saturation du sol par le fluide, c'est-à-dire jusqu'au point, où ledit volume varie à nouveau obligatoirement avec ladite pression;
- on calcule la différence de pression mesurée entre les points, correspondant à la pression de gonflement du sol.

A partir du point précédent de remise du sol à son état initial et de l'apport du fluide dans ce sol autour de la sonde, on peut, suivant un autre procédé de l'invention, contrôler la pression dans ladite sonde, afin qu'elle reste constante en diminuant alors le volume de celle-ci jusqu'à saturation du sol par le fluide, c'est-à-dire jusqu'au point où ladite pression varie obligatoirement à nouveau avec ledit volume et calculer la différence de volume mesurée entre les points, correspondant à la dilatation libre due au gonflement du sol.

Enfin, l'objectif principal de l'invention est atteint quand on effectue lesdits calculs de pression de gonflement du sol à volume constant et de dilatation du sol à pression constante successivement l'une après l'autre, en se déplaçant le long des courbes de variation de pression en fonction du volume pour un sol saturé de fluide, en mesurant les valeurs de pression et de volume entre les points de ces courbes correspondant soit au volume, soit à la pression du point de remise du sol à son état initial, avant imbibition.

Une autre solution au problème posé est un dispositif de mesure in situ des caractéristiques de gonflement d'un sol comprenant, d'une manière connue, une sonde dilatable, des moyens pour introduire et dilater celle-ci dans le sol et des moyens pour contrôler la pression radiale exercée sur le sol par la sonde; lequel dispositif comporte des moyens d'injection pour apporter dans le sol entourant au moins une partie de ladite sonde, un fluide qui imbibe le sol, sous une charge faible correspondant à quelques mètres de colonne de ce fluide, ce dispositif, dans un mode de réalisation préférentiel comporte au moins deux parties gonflables indépendamment l'une de l'autre, l'une permettant des mesures normales et classiques de pression de sol dans son état initial, l'autre associée audit moyen d'injection du fluide permettant des mesures de pression de gonflement du sol simultanément à l'imbibition de celui-ci jusqu'à saturation, soit à pression constante, soit à volume constant.

Le résultat est de nouveaux procédés et dispositifs de mesure in situ des caractéristiques de gonflement d'un sol. Ces procédés et dispositifs apportent de nombreux avantages par rapport aux techniques actuelles, dont, il faut le répéter, aucune ne permet à ce jour de mensurer in situ les caractéristiques recherchées de gonflement, celles-ci étant effectuées en laboratoire, donc en temps différé avec les inconvénients cités précédemment.

De plus, l'adaptation de la présente invention sur des matériels de mesure existants permet, d'une part, de réduire le coût de mise en oeuvre et d'investissement, mais, d'autre part, de recaler les mesures faites par rapport aux étalonnages connus effectués justement avec ce matériel. En effet, les caractéristiques de mécanique des sols ne sont souvent pas des mesures absolues mais surtout relatives, et il est donc nécessaire et important d'avoir les mêmes références de mesure de base, surtout que c'est à partir de certaines de celles-ci que des normes ont été établies en matière de construction de bâtiment.

De plus, le profil pressiométrique classique peut ensuite être continué dans le même forage à plus grande profondeur, en dehors de la zone de gonflement potentiel.

Dans des modes préférentiels de réalisations, la durée de l'essai dit "sec" de référence avec une sonde en deux parties gonflables indépendantes est mis à profit pour réaliser le gonflement, car le temps d'imbibition du sol correspond à peu près à la durée de l'essai dit "sec" précédent, soit de l'ordre de 15 minutes; ceci permet de gagner du temps d'intervention.

Enfin, des améliorations et des capteurs supplémentaires peuvent être insérés dans la sonde pour suivre par exemple la pression interstitielle.

Dans le descriptif suivant, nous décrivons essentiellement des exemples de procédés et de dispositifs suivant l'invention, mais d'autres outils et sondes peuvent être envisagés dans le cadre de celle-ci : les dessins, figures et descriptif n'ont aucun caractère limitatif.

La figure 1 est une représentation des courbes des valeurs de pression et de volume.

La figure 2 est une vue en coupe d'ensemble du dispositif de mesure.

La figure 3 est une vue simplifiée en perspective de la sonde en deux parties gonflables superposées.

La figure 4 est une vue simplifiée en perspective de la sonde en quatre secteurs gonflables.

Les figures 5A et 5B sont des représentations en plan de dessus de la déformation d'un sol par la sonde à quatre secteurs.

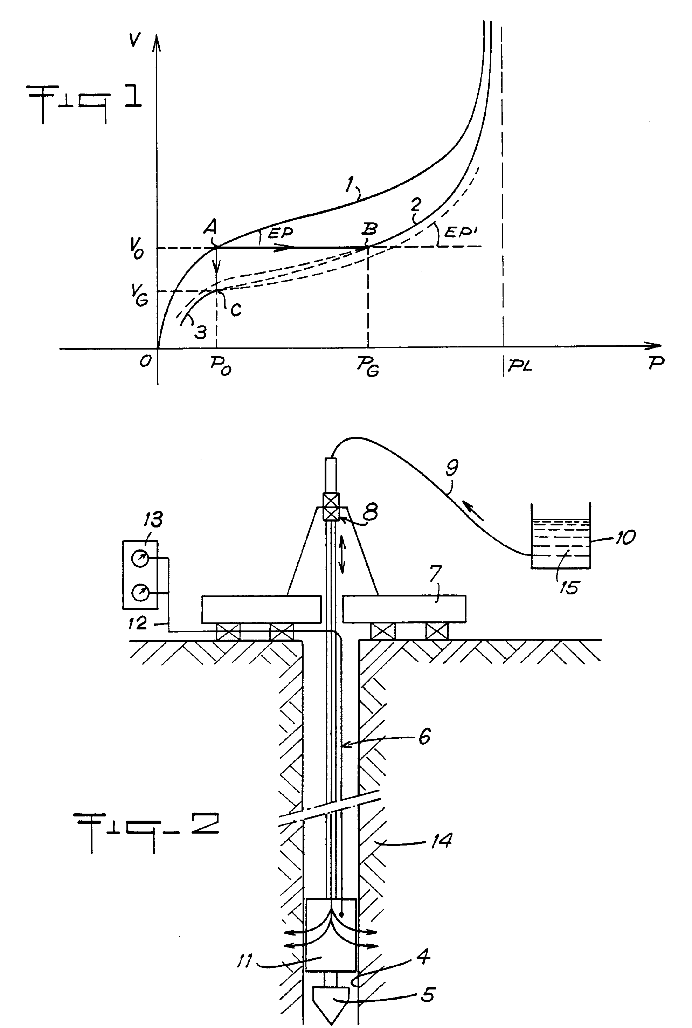

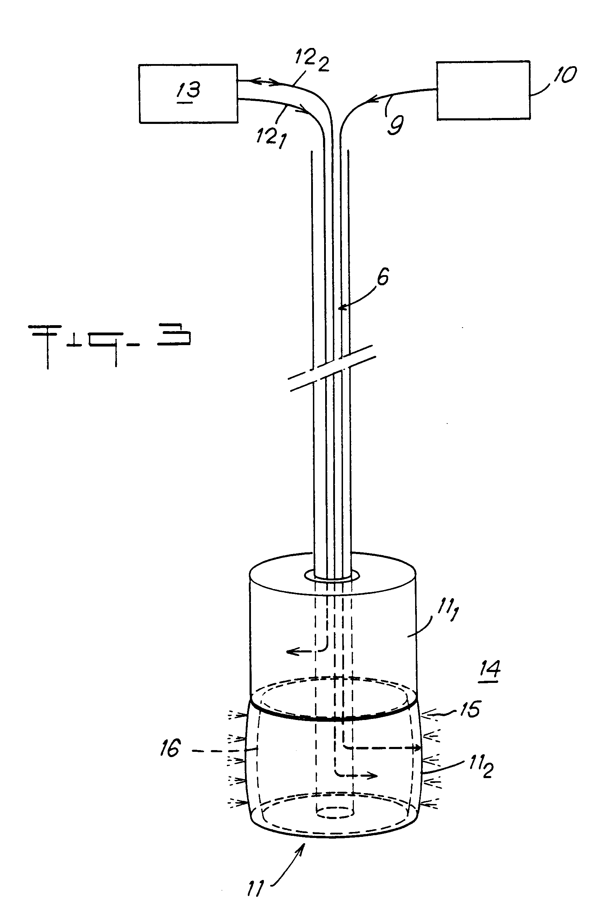

La figure 1 représente un repère d'axes orthogonaux dont les abscisses (P) représentent les valeurs de pression dans une sonde gonflable, après un forage de trou dans un sol et introduction de cette sonde ayant un diamètre extérieur à peu près égal au trou effectué, en général par exemple de l'ordre de 63 mm pour les appareils existants, et les ordonnées (V) représentent les valeurs de volume de cette sonde. Ces valeurs sont mesurées en surface tel que représenté dans la figure 2.

En opération, l'utilisateur mesure et relève une courbe 1 naturelle pression-volume, qu'il utilise ensuite pour déterminer manuellement les caractéristiques du sol, dont un module pressiométrique normal EP et une pression PL limite conventionnelle (valeurs dont la signification est liée à des données de base sur l'appareil "Pressiomètre"). En règle générale, il est procédé d'abord à un étalonnage, la sonde étant à l'air libre, à peu près au niveau du dispositif de surface; ensuite, avec la sonde enterrée dans un forage, sont effectuées les mesures proprement dites, d'où sont soustraites les valeurs d'étalonnage, pour éliminer la réponse propre de la sonde.

Le module "pressiométrique" est la pente de la courbe 1 au point A correspondant à la remise en place du sol à son état initial avant forage.

Le point A donne donc la pression P₀ et le volume V₀ initial qui caractérisaient la partie du sol dont la sonde a pris la place.

Une fois la sonde selon l'invention, et telle que décrite dans la figure suivante, mise en place dans le trou de forage à l'endroit désiré et positionnée en valeurs de pression et volume pour correspondre à ce point A, on apporte dans le sol entourant au moins une partie de la sonde, un fluide qui imbibe le sol sous une charge faible, correspondant à quelques mètres de colonnes de ce fluide.

Divers procédés de mesures sont alors possibles :
a/ soit on contrôle en même temps que cet apport de fluide dans le sol le volume de la sonde pour qu'il reste constant, en augmentant alors la pression dans celle-ci jusqu'à saturation du sol par le fluide, c'est-à-dire en parcourant le segment de droite AB sur la figure jusqu'au point B où ledit volume V ne peut alors qu'augmenter avec ladite pression P, permettant alors de tracer la courbe 2. Cette courbe représente alors la phase d'expansion de la sonde après le gonflement du terrain à saturation : la pression limite PL n'est à priori pas affectée par l'imbibition du sol tandis que le module Ep est affecté. La différence de pression mesurée entre les points B et A soit δP = P_{G} - P₀ correspond alors à la pression de gonflement du sol.
b/ soit on contrôle en même temps que cet apport de fluide la pression dans la sonde, pour qu'elle reste constante et égale à P₀ en diminuant alors son volume jusqu'à saturation du sol par le fluide, c'est-à-dire en parcourant le segment AC sur la figure jusqu'au point C où ladite pression ne peut alors que diminuer avec le volume permettant de tracer la courbe 3. Cette dernière courbe, si on la prolonge en réaugmentant la pression P et donc le volume V, doit normalement se confondre avec la courbe 2 précédente et passer par le point B, car il s'agit de la courbe pression-volume du sol saturé.
   La différence des volumes mesurées entre les points A et C, soit δV = V₀ - V_{G} correspond au volume de dilatation libre dû au gonflement du sol.
c/ soit si on ne veut pas faire deux mesures différentes en deux points différents, ce qui risquerait de ne pas être concordant, on peut mesurer la pression de gonflement en parcourant le segment AB comme indiqué ci-dessus en a/, puis une fois l'apport de fluide réalisé à saturation du sol, on fait chuter la pression P_{G} dans la sonde en parcourant alors la courbe 2 ou 3, jusqu'à obtenir la pression P₀ initiale en revenant au point C, pour lequel on mesure le volume V_{G} de la sonde, permettant de calibrer le volume de dilatation libre δV comme indiqué ci-dessus en b/.
d/ soit on peut mesurer d'abord le volume de dilatation libre δV comme ci-dessus en b/, puis en augmentant la pression dans la sonde, remonter au point B et mesurer la pression de gonflement comme en a/ ci-dessus; les procédés c/ et d/ sont équivalents quant à la définition de cette pression de gonflement.

La figure 2 est une vue en coupe d'ensemble du dispositif de mesure qui comprend un support connu et calé sur le sol 14, permettant à partir de tout type de moyen de forage 8 de réaliser un trou de forage 4 à l'endroit et à la profondeur où l'on veut effectuer la mesure.

Dans un mode de réalisation préférentiel, on utilise des équipements tels que par exemple ceux des appareils appelés suivant la marque déposée "Pressiomètre", qui comportent, en outre, un appareillage 13 connu, permettant de contrôler la pression et le volume de toute sonde déformable 11 qui lui est reliée par au moins un conduit 12.

La sonde 11 suivant l'invention, tel que décrit dans la figure suivante, est reliée en fait par deux conduits à un appareillage 13 qui est alors doublé.

La sonde munie éventuellement d'une pointe de battage 5, est introduite et descendue dans le trou de forage par un train de tiges 6 jusqu'à la profondeur où l'on veut effectuer la mesure.

De plus, un réservoir 10 contenant un fluide quelconque 15 est relié également à ladite sonde 11 par un conduit 9 passant ou non dans le train de tige 6, comme ceux 12 reliés au dispositif de mesure 13. Ce fluide 15, au contraire de celui utilisé pour dilater la sonde et qui est donc d'un volume déterminé et récupérable, est perdu et utilisé pour imbiber une partie au moins du sol 14 autour de la sonde 11, dans lequel il est injecté sous une charge faible correspondant à quelques mètres de colonne de ce fluide. Son injection peut ainsi être faite par simple gravité, et ce fluide est de préférence de l'eau.

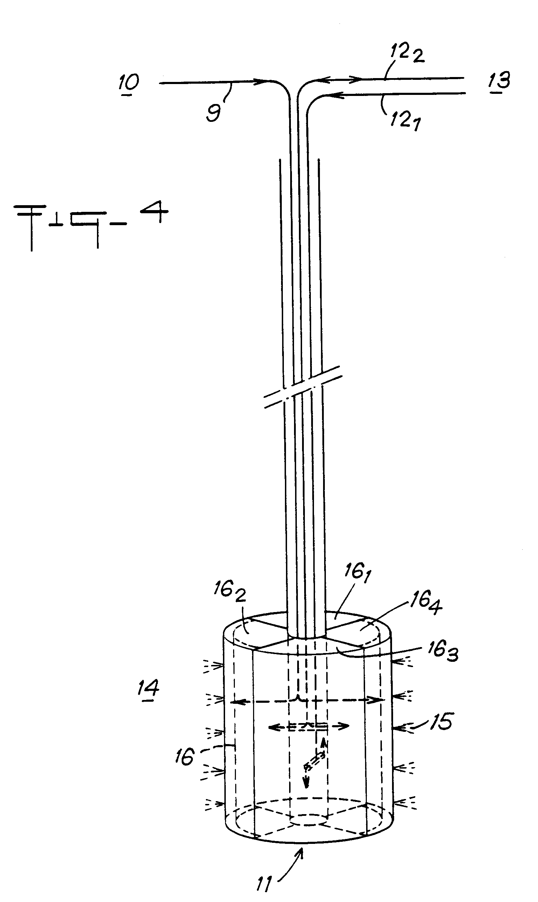

La figure 3 est une vue simplifiée d'un exemple de ladite sonde 11 constituée, afin d'obtenir le meilleur résultat de mesure suivant le procédé, de deux parties gonflables indépendamment l'une de l'autre permettant d'effectuer les mesures suivant l'un des procédés de l'invention décrits dans la figure 1, en particulier, des mesures simultanées de pression normale et de pression de gonflement.

Dans cette figure, les deux parties sont en fait deux sondes superposées gonflables : la partie supérieure 11₁, alimentée par un conduit 12₁ depuis l'appareillage 13 à travers par exemple le train de tige 6 est de type connu et permet des mesures normales et classiques de pression de sol dans son état initial; la partie inférieure 11₂ est elle-aussi alimentée par un conduit 12₂ depuis l'appareillage 13 pour permettre des mesures de pression du sol autour d'elle comme la précédente, mais de plus, elle est associée audit réservoir 10 de fluide 15 par le conduit 9 : le fluide peut alors être injecté dans le sol 14 au travers d'une double paroi 16 par exemple, entourant tout ou partie de la sonde inférieure 11₂ sur sa périphérie : la paroi intérieure étanche de celle-ci agit alors comme transmetteur de pression pour la mesure, comme la paroi d'une sonde gonflable, et la paroi externe poreuse comportant des orifices n'induit aucune pression parasite pouvant influencer les mesures quand il y a équilibre de saturation d'imbibition du sol par le fluide 15 et que celui-ci ne peut plus être injecté.

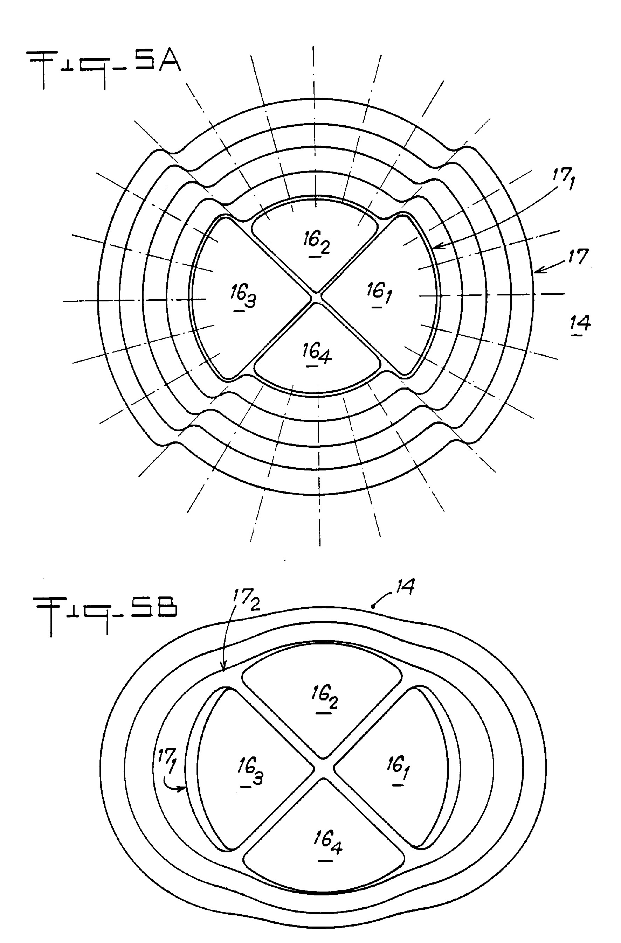

La figure 4 est une vue simplifiée d'un autre exemple de ladite sonde 11, constitué également de deux parties gonflables indépendamment l'une de l'autre, dont chaque partie est elle-même dédoublée, de telle façon que la sonde 11 est en fait constituée d'au moins quatre secteurs 16₁, 16₂, 16₃ et 16₄ gonflables couplés deux à deux en opposition et travaillant alors dans la même couche du sol 14, ce qui peut être préférable.

La partie constituée des secteurs 16₁ et 16₃ est reliée par un conduit 12₁ pour les mesures de pressions normales et classiques de pressions depuis l'appareillage 13. La partie constituée des secteurs 16₂ et 16₄ est reliée par un conduit 12₂ à cet appareillage pour les mesures de gonflement et, de plus, elle est reliée au réservoir 10 de fluide par le conduit 9 pour l'injection de ce fluide 15 dans le sol, à travers par exemple comme dans la figure précédent, une double paroi 16 recouvrant la seule surface externe des secteurs 16₂ et 16₄.

Dans une autre variante, les secteurs peuvent être recouverts chacun d'une paroi rigide extérieure, dont les deux recouvrant les secteurs 16₂ et 16₄ comprennent des canaux et orifices à travers lesquels passe le fluide amené directement par le conduit 9, en plan.

Les figures 5A et 5B sont des représentations de dessus ou de dessous de la déformation d'un sol 14 par la sonde décrite dans la figure 4 précédente. Chaque ligne 17 correspond à un isobare.

Dans la figure 5A, le couple de secteurs 16₂ et 16₄ est représenté ici après l'injection de fluide dans le sol dans la position du point C de la figure 1, alors que les secteurs 16₁ et 13₃ sont dans la position de pression normale du sol 14, soit au point A de la figure 1. La première ligne de pression 17₁ correspond donc à la pression P₀ et la différence de volume mesurable entre le couple des secteurs (16₁ + 16₃) et (16₂+ 16₄) représente la variation de dilatation du sol V_{G} - V₀.

Dans la figure 5B, le couple des secteurs 16₂ et 16₄, toujours représentés après injection de fluide dans le sol 14, est ici ramené au même volume que les secteurs 16₁ et 16₃ avec donc une périphérie globale de la sonde en forme de cercle : ainsi, tous les secteurs correspondent au volume du terrain reconstitué dans sa position initiale avant forage. Les secteurs 16₁ et 16₃ sont toujours dans la position de pression normale sur un point A de la figure 1, et les secteurs 16₂ et 16₄ sont au point B de cette figure.

La première ligne de pression 17₁ correspondant à la pression P₀, s'arrête donc près des extrémités des secteurs 16₁ et 16₃, alors que la ligne 17₂ suivant la paroi externe des secteurs 16₂ et 16₄ correspond à la pression P_{G} de gonflement et s'écarte ensuite des secteurs 16₁ et 16₃. La différence de pression P_{G} - P₀ est la pression de gonflement du sol.

## Revendications

1. Procédé de mesure in situ des caractéristiques de gonflement d'un sol à partir d'un équipement comprenant une sonde dilatable (11), des moyens (8) pour introduire cette sonde dans le sol et la dilater et des moyens (13) pour contrôler la pression radiale exercée sur le sol (14) par la sonde, caractérisé en ce que :
- on introduit dans un forage (4) réalisé dans le sol (14) à étudier ladite sonde (11) dilatable à la profondeur voulue et on réalise un essai pressiométrique normal connu, consistant à établir la courbe (1) de variation de la pression (P) de la sonde et donc dans le sol autour de celle-ci, en fonction du volume (V) de dilatation de cette sonde et donc du volume comprimé du sol;
- on apporte dans ledit sol (14) entourant au moins une partie de la sonde (11) et à partir du point (A) de remise du sol à son état initial, comme s'il n'y avait pas de trou de forage, un fluide (15) qui imbibe le sol, sous une charge faible correspondant à quelques mètres de colonne de ce fluide;
- on contrôle simultanément le volume (V) de ladite sonde afin qu'il reste constant en augmentant alors la pression (P) dans celle-ci, jusqu'à saturation du sol par le fluide (15), c'est-à-dire jusqu'au point (B), où ledit volume varie à nouveau obligatoirement avec ladite pression;
- on calcule la différence de pression (δP) mesurée entre les points (B et A), correspondant à la pression degonflement du sol.

2. Procédé de mesure in situ des caractéristiques de gonflement d'un sol à partir d'un équipement comprenant une sonde dilatable (11), des moyens (8) pour introduire cette sonde dans le sol et la dilater et des moyens (13) pour contrôler la pression radiale exercée sur le sol (14) par la sonde, caractérisé en ce que :
- on introduit dans un forage (4) réalisé dans le sol (14) à étudier ladite sonde (11) dilatable à la profondeur voulue et on réalise un essai pressiométrique normal connu, consistant à établir la courbe (1) de variation de la pression (P) de la sonde et donc dans le sol autour de celle-ci, en fonction du volume (V) de dilatation de cette sonde et donc du volume comprimé du sol;
- on apporte dans ledit sol (14) entourant au moins une partie de la sonde (11) et à partir du point (A) de remise du sol à son état initial, comme s'il n'y avait pas de trou de forage, un fluide (15) qui imbibe le sol, sous une charge faible correspondant à quelques mètres de colonne de ce fluide;
- on contrôle simultanément la pression (P) dans ladite sonde (11), afin qu'elle reste constante en diminuant alors le volume (V) de celle-ci jusqu'à saturation du sol par le fluide (15), c'est-à-dire jusqu'au point (C) où ladite pression varie obligatoirement à nouveau avec ledit volume;
- on calcule la différence de volume (V) mesurée entre les points (A et C), correspondant à la dilatation libre due au gonflement du sol.

3. Procédé suivant l'une quelconque des revendications 1 et 2, caractérisé en ce que l'on effectue lesdits calculs de pression de gonflement du sol à volume constant (V₀) et de dilatation du sol à pression constante (P₀) successivement l'un après l'autre, en se déplaçant le long des courbes (2 et 3) variations de pression en fonction du volume pour un sol saturé de fluide (15), en mesurant les valeurs de pression (P) et de volume (V) entre les points de ces courbes correspondant soit au volume, soit à la pression du point de remise du sol (14) à son état initial, avant imbibition.

4. Dispositif de mesure in situ des caractéristiques de gonflement d'un sol comprenant, d'une manière connue, une sonde dilatable (11), des moyens (8) pour introduire et dilater celle-ci dans le sol (14) et des moyens (13) pour contrôler la pression radiale (P) exercée sur le sol par la sonde (11), caractérisé en ce qu'il comporte des moyens (10) d'injection pour apporter dans le sol (14) entourant au moins une partie de ladite sonde (11), un fluide (15) qui imbibe le sol, sous une charge faible correspondant à quelques mètres de colonne de ce fluide.

5. Dispositif suivant la revendication (4), caractérisé en ce que ladite sonde (11) comporte au moins deux parties gonflables indépendamment l'une de l'autre, l'une (11₁) permettant des mesures normales et classiques de pression de sol dans son état initial, l'autre (11₂) associée audit moyen (10) d'injection du fluide permettant des mesures de pression de gonflement du sol simultanément à l'imbibition de celui-ci jusqu'à saturation, soit à pression constante, soit à volume constant.

6. Dispositif suivant la revendication 5, caractérisé en ce que ladite sonde (11) est constituée de deux sondes superposées gonflables.

7. Dispositif suivant la revendication 5, caractérisé en ce que ladite sonde (11) est constituée d'au moins quatre secteurs gonflables (16) couplés deux à deux en opposition et travaillant dans la même couche de sol (14).

8. Dispositif suivant l'une quelconque des revendications 4 à 7, caractérisé en ce que ledit fluide (15) est de l'eau.

## Patentansprüche

1. Verfahren zur In-situ-Messung der Quelleigenschaften eines Bodens mittels einer Ausrüstung, die eine dehnbare Sonde (11), Einrichtungen (8) zum Einführen dieser Sonde in den Boden und zum Dehnen derselben und Einrichtungen (13) zur Kontrolle des auf den Boden (14) durch die Sonde ausgeübten radialen Drucks umfaßt,
dadurch **gekennzeichnet,** daß:
- die dehnbare Sonde (11) in ein Bohrloch (4) im zu untersuchenden Boden (14) in die gewünschte Tiefe abgesenkt wird und ein an sich bekannter Druckmeßversuch durchgeführt wird, der darin besteht, die Kurve (1) der Veränderung des Drucks (P) der Sonde und damit des Bodens um diese herum in Abhängigkeit vom Dehnungsvolumen (V) dieser Sonde und damit des komprimierten Volumens des Bodens aufgenommen wird;
- ausgehend vom Punkt (A), der dem ursprünglichen Zustand des Bodens ohne Bohrloch entspricht, in den Boden (14) ein Fluid (15) mit geringem Druck entsprechend einer Fluidsäule von wenigen Metern Höhe eingebracht und der Boden mit dem Fluid durchtränkt wird;
- gleichzeitig das Volumen (V) der Sonde kontrolliert wird, so daß es unter Zunahme des Drucks (P) in derselben bis zur Sättigung des Bodens mit dem Fluid (15) konstant bleibt, das heißt bis zum Punkt (B), an dem sich das Volumen wieder zwangsläufig mit dem Druck ändert;
- die Differenz (δP) zwischen den an den Punkten (B und A) gemessenen Drücken berechnet wird, die dem Quelldruck des Bodens entspricht.

2. Verfahren zur In-situ-Messung der Quelleigenschaften eines Bodens mittels einer Ausrüstung, die eine dehnbare Sonde (11), Einrichtungen (8) zum Einführen dieser Sonde in den Boden und zum Dehnen derselben und Einrichtungen (13) zur Kontrolle des auf den Boden (14) durch die Sonde ausgeubten radialen Drucks umfaßt,
dadurch gekennzeichnet, daß
- die dehnbare Sonde (11) in ein Bohrloch (4) im zu untersuchenden Boden (14) in die gewünschte Tiefe abgesenkt wird und ein an sich bekannter Druckmeßversuch durchgeführt wird, der darin besteht, die Kurve (1) der Veränderung des Drucks (P) der Sonde und damit des Bodens um diese herum in Abhängigkeit vom Dehnungsvolumen (V) dieser Sonde und damit des komprimierten Volumens des Bodens aufgenommen wird;
- ausgehend vom Punkt (A), der dem ursprünglichen Zustand des Bodens ohne Bohrloch entspricht, in den Boden (14) ein Fluid (15) mit geringem Druck entsprechend einer Fluidsäule von wenigen Metern Höhe eingebracht und der Boden mit dem Fluid durchtränkt wird;
- gleichzeitig der Druck (P) in der Sonde (11) kontrolliert wird, so daß dieser konstant bleibt, während das Volumen (V) derselben bis zur Sättigung des Bodens mit dem Fluid (15) vermindert wird, das heißt bis zum Punkt (C), an dem der Druck sich wieder zwangsläufig mit dem Volumen ändert;
- die Differenz (δV) zwischen den an den Punkten (A und C) gemessenen Volumina berechnet wird, die der freien Dehnung aufgrund der Quellung des Bodens entspricht.

3. Verfahren nach einem der Ansprüche 1 oder 2,
dadurch gekennzeichnet, daß
die Messungen des Quelldrucks des Bodens bei konstantem Volumen (V₀) und der Dehnung des Bodens bei konstantem Druck (P₀) nacheinander durchgeführt werden, indem Druck- und Volumenänderungen entlang der Kurven (2 und 3) für einen fluidgesättigten Boden durchlaufen werden und die Werte des Drucks (P) und des Volumens (V) zwischen denjenigen Punkten dieser Kurven gemessen werden, die entweder dem Volumen oder dem Druck des Ursprungszustands des Bodens (14) vor seiner Durchtränkung entsprechen.

4. Vorrichtung zur In-situ-Messung der Quelleigenschaften eines Bodens, mit einer dehnbaren Sonde (11), Einrichtungen (8) zum Einführen und Dehnen derselben im Boden (14) und Einrichtungen (13) zur Kontrolle des durch die Sonde (11) auf den Boden ausgeübten radialen Drucks (P),
gekennzeichnet durch
Einspritzeinrichtungen (10) zum Eintragen eines Fluids (15), das den Boden durchtränkt, in den die Sonde (11) wenigstens teilweise umgebenden Boden (14) unter geringem Druck entsprechend wenigen Metern Fluidsäule.

5. Vorrichtung nach Anspruch 4,
dadurch gekennzeichnet, daß
die Sonde (11) wenigstens zwei unabhängig voneinander dehnbare Teile umfaßt, von denen eines (11₁) normale herkömmliche Druckmessungen des Bodens in seinem Ursprungszustand ermöglicht und das mit der Fluideinspritzeinrichtung (10) verbundene andere Teil (11₂) gleichzeitig mit der Durchtränkung des Bodens bis zur Sättigung Messungen des Quelldrucks des Bodens bei konstantem Druck oder bei konstantem Volumen ermöglicht.

6. Vorrichtung nach Anspruch 5,
dadurch gekennzeichnet, daß
die Sonde (11) aus zwei übereinander angeordneten dehnbaren Sonden besteht.

7. Vorrichtung nach Anspruch 5,
dadurch gekennzeichnet, daß
die Sonde (11) aus mindestens vier dehnbaren Sektoren (16) besteht, die paarweise gegenständig gekoppelt sind und in derselben Bodenschicht (14) arbeiten.

8. Vorrichtung nach einem der Ansprüche 4 bis 7,
dadurch gekennzeichnet, daß
das Fluid (15) Wasser ist.

## Claims

1. Method for measuring in-situ the ground heave characteristics from an equipment comprising an expansible sensor (11), means (8) for introducing this sensor in the ground and expanding it and means (13) for monitoring the radial pressure exerted on the ground (14) by the sensor, characterized in that:
- said expansible sensor (11) is introduced to the desired depth in a bore-hole (4) made in the ground (14) to be studied and a known normal pressiometric test is made, consisting in establishing the curve (1) of variation of the pressure (P) of the sensor and therefore in the ground therearound, as a function of the volume (V) of expansion of this sensor and therefore of the compressed volume of the ground;
- a fluid (15) is supplied in said ground (14) surrounding at least a part of the sensor (11) and from point (A) of return of the ground to its initial state, as if there were no bore-hole, which fluid impregnates the ground, under a low charge corresponding to some metres of column of this fluid;
- the volume (V) of said sensor is simultaneously monitored so that it remains constant, in that case increasing the pressure (P) therein, up to saturation of the ground by the fluid (15), i.e. up to point (B), where said volume again varies compulsorily with said pressure;
- the difference in pressure (δP) measured between the points (B and A), corresponding to the ground heave pressure, is calculated.

2. Method of measuring in-situ the ground heave characteristics from an equipment comprising an expansible sensor (11), means (8) for introducing this sensor into the ground and for expanding it and means (13) for monitoring the radial pressure exerted on the ground (14) by the sensor, characterized in that:
- said expansible sensor (11) is introduced to the desired depth in a bore-hole (4) made in the ground (14) to be studied, and a known normal pressiometric test is made, consisting in establishing the curve (1) of variation of the pressure (P) of the sensor and therefore in the ground therearound, as a function of the volume (V) of expansion of this sensor and therefore of the compressed volume of the ground;
- a fluid (15) is supplied in said ground (14) surrounding at least a part of the sensor (11) and from point (A) of return of the ground to its initial state, as if there were no bore-hole, which fluid impregnates the ground, under a low charge corresponding to some metres of column of this fluid;
- the pressure (P) in said sensor (11) is simultaneously monitored so that it remains constant, in that case reducing the volume (V) thereof up to saturation of the ground by the fluid (15), i.e. up to point (C) where said pressure again varies compulsorily with said volume;
- the difference in volume (V) measured between points (A and C), corresponding to the free expansion due to the ground heave, is calculated.

3. Method according to either one of Claims 1 and 2, characterized in that said calculations of ground heave pressure at constant volume (V_{O}) and of expansion of the ground at constant pressure (P_{O}) are effected successively one after the other, by moving along the curves (2 and 3) of variations of pressure as a function of the volume for ground saturated with fluid (15), by measuring the values of pressure (P) and of volume (V) between the points of these curves corresponding either to the volume, or to the pressure of the point of return of the ground (14) to its initial state, before impregnation.

4. Device for measuring in-situ the ground heave characteristics, comprising, in known manner, an expansible sensor (11), means (8) for introducing and expanding the latter in the ground (14) and means (13) for monitoring the radial pressure (P) exerted on the ground by the sensor (11), characterized in that it comprises injection means (10) for supplying in the ground (14) surrounding at least a part of said sensor (11), a fluid (15) which impregnates the ground, under a low charge corresponding to some metres of column of this fluid.

5. Device according to Claim 4, characterized in that said sensor (11) comprises at least two parts inflatable independently of each other, one (11₁) allowing standard conventional measurements of ground pressure in its initial state, the other (11₂) associated with said fluid injection means (10) allowing measurements of ground heave pressure simultaneously with the impregnation thereof up to saturation, either at constant pressure, or at constant volume.

6. Device according to Claim 5, characterized in that said sensor (11) is constituted by two superposed inflatable sensors.

7. Device according to Claim 5, characterized in that said sensor (11) is constituted by at least four inflatable sectors (16) coupled in two's in opposition and working in the same strata of ground (14).

8. Device according to any one of Claims 4 to 7, characterized in that said fluid (15) is water.
